# EUROPEAN PATENT SPECIFICATION

(11) **EP 1 402 883 B1**
(45) Date of publication and mention of the grant of the patent: **19.04.2006**
(21) Application number: 03021682.4
(22) Date of filing: 29.09.2003
(51) Int. Cl.: A61K 8/37, A61K 8/27, A61K 8/29, A61K 8/60, A61Q 17/04

(54) **Octyl methoxycinnamate containing skin preparation**
Hautpflegemittel enthaltend Octylmethoxycinnamat
Composition pour la peau comprenant de l'octyl methoxycinnamate

(30) Priority: 30.09.2002 JP 2002285382
(43) Date of publication of application: 31.03.2004
(73) Proprietor: Shiseido Co., Ltd., Tokyo 104-0061 (JP)
(72) Inventor: Takafumi, Kurosawa, c/o Shiseido Research Center, Yokohama-shi Kanagawa 224-8558 (JP); Shoichro, Shio, c/o Shiseido Research Center, Yokohama-shi Kanagawa 224-8558 (JP); Hiroshi, Itagaki, 2-12-1 Fukuura Kanazawa-ku, 236-8643 (JP); Hirokazu, Kouzuki, 2-12-1 Fukuura Kanazawa-ku, 236-8643 (JP)
(74) Representative: Merkle, Gebhard

(56) References cited:
- WO-A-00/33803
- US-B1- 6 294 156
- PATENT ABSTRACTS OF JAPAN vol. 2002, no. 11, 6 November 2002 (2002-11-06) & JP 2002 212024 A (SHISEIDO CO LTD), 31 July 2002 (2002-07-31)
- PATENT ABSTRACTS OF JAPAN vol. 013, no. 427 (C-639), 22 September 1989 (1989-09-22) & JP 01 165517 A (SEIHO:KK;OTHERS: 01), 29 June 1989 (1989-06-29)

## Description

### Related Application

This application claims the priority of Japanese application No.2002-285382 filed on 30^{th} September,2002, which is incorporated herein by reference.

### Field of the Invention

The present invention relates to an external skin preparation with an effect of reducing irritation. More specifically, the invention relates to an external skin preparation prepared by blending polyoxyethylene methyl glucoside and/or polyoxypropylene methyl glucoside in an external skin preparation containing octyl methoxycinnamate and titanium oxide and/or zinc oxide to reduce the irritation of octyl methoxycinnamate on the skin.

### Background of the Invention

Ultraviolet absorbents are blended in external skin preparations primarily including sun-screening cosmetics. Particularly, powders of titanium oxide, zinc oxide as ultraviolet reflectors are commonly blended together with an ultraviolet absorbent octyl methoxycinnamate in sun-screening cosmetics (JP-A-7-267842, patent reference 1).

Meanwhile, users with sensitive skin may sometimes feel skin irritation from octyl methoxycinnamate in sun-screening cosmetics.

Therefore, a method for reducing the irritation has been sought (JP-A-2002-212024, patent reference 2). This patent reference 2 describes a method for reducing the skin irritation due to ultraviolet absorbents by blending polyethylene glycol. It also describes in Examples 1 and 2 therein an external skin preparation and a sun-screening cosmetic containing octyl methoxycinnamate, zinc oxide in fine particle and titanium dioxide. The irritation is reduced by polyethylene glycol.

### Summary of the Invention

From such standpoint, the present inventors have made investigations about a method for reducing the irritation of ultraviolet absorbents so as to obtain a sun-screening cosmetic with low skin irritation. The inventors have found that a given amount of octyl methoxycinnamate when blended in an external skin preparation never causes skin irritation but the external skin preparation once blended with powders of titanium oxide and zinc oxide causes skin irritation; and that the external skin preparation when additionally blended with polyoxyethylene methyl glucoside and/or polyoxypropylene methyl.glucoside can reduce the skin irritation. Thus, the invention has been achieved.

In other words, the invention provides an external skin preparation containing octyl methoxycinnamate, titanium oxide and/or zinc oxide, polyoxyethylene methyl glucoside and/or polyoxypropylene methyl glucoside.

Additionally, the invention provides a sun-screening cosmetic, which is the external skin preparation.

Still additionally, the invention provides an irritation-reducing agent containing polyoxyethylene methyl glucoside and/or polyoxypropylene methyl glucoside which reduce the irritation of octyl methoxycinnamate in an external skin preparation containing titanium oxide and/or zinc oxide.

Further, the invention provides a method for reducing the skin irritation of octyl methoxycinnamate, including blending polyoxyethylene methyl glucoside and/or polyoxypropylene methyl glucoside in an external skin preparation containing octyl methoxycinnamate and titanium oxide and/or zinc oxide.

### Brief Description of the Drawing

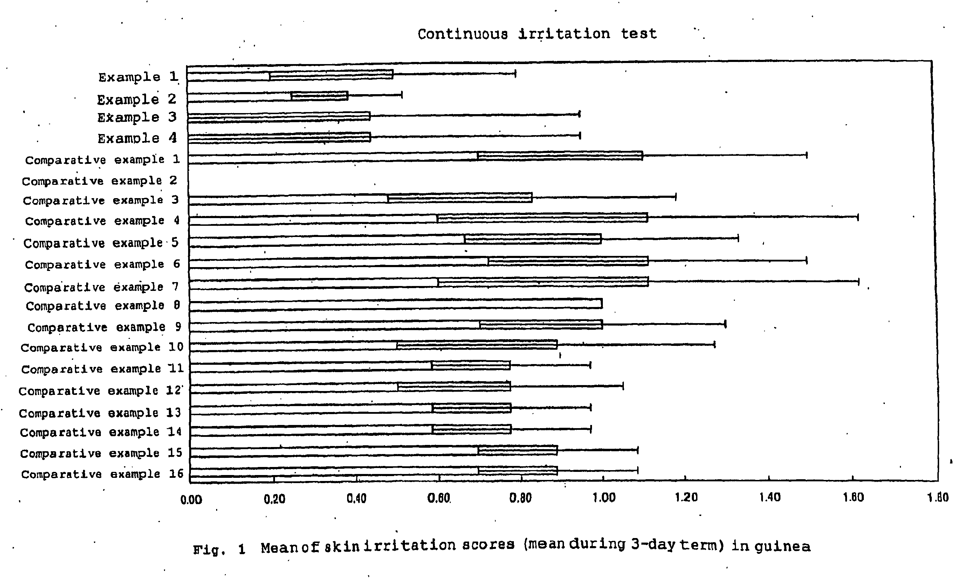

Fig.1 depicts graphs showing the results of a continuous skin irritation test.

### Detailed Description of the Invention

The invention relates to an external skin preparation with an irritation-reducing effect, is produced by blending polyoxyethylene methyl glucoside and/or polyoxypropylene methyl glucoside in an external skin preparation containing octyl methoxycinnamate, powdered titanium oxide and/or zinc oxide, to reduce skin irritation caused by the octyl methoxycinnamate.

The invention is now described in detail hereinbelow.

The external skin preparation of the invention is blended with octyl methoxycinnamate as an ultraviolet absorbent. A great number of currently commercially available products of octyl methoxycinnamate can be used. For example, octyl methoxycinnamate is commercially available under the trade name PARSOL MCX (Givaudan Company).

The amount of octyl methoxycinnamate to be blended is appropriately determined, depending on the intended SPF value for designing an external skin product. Generally, octyl methoxycinnamate is blended at about 1.0 to 15 % by weight, preferably at 2.0 to 10 % by weight.

Because powdered titanium oxide and/or zinc oxide functions as an ultraviolet reflector, the titanium oxide and/or zinc oxide is frequently blended together with octyl methoxycinnamate in sun-screening cosmetics. The inventors have found that octyl methoxycinnamate with skin irritant activity.does not necessarily cause the irritation when it is at 10 % or less in external skin preparations but causes the irritation when blended with the powders. These powders possibly have an action to enhance the irritation of octyl methoxycinnamate.

In accordance with the invention, commercially available powdery products of titanium oxide and/or zinc oxide can be used as the powdered titanium oxide and/or zinc oxide.

As the powders, generally, needle-like, spindle-like, sphere-like and grain-like such powders are used. Further, fine particle powders of a particle size of 0.1 µm or less are preferable.

Additionally when such powders are treated in a hydrophobic manner with silicone processing with methyl hydrogen polysiloxane and silane coupling agents, metal soap processing, fluorine processing with perfluoroalkylphosphate diethanolamine salt and perfluoroalkylsilane and processing with dextrin fatty acid ester, the resulting powders are preferred.

The amount of the powders to be blended is not specifically limited. Generally, the amount is appropriately determined within a range of 1.0 to 40 % (percentage by weight) to the total amount of an external skin preparation. In view of the intended SPF value, usability and stability, the amount is preferably at 3.0 to 25 % (percentage by weight) to the total amount of a sun-screening emulsified cosmetic.

In accordance with the invention, polyoxyethylene methyl glucoside and/or polyoxypropylene methyl glucoside functions as an irritation-reducing agent for octyl methoxycinnamate.

Polyoxyethylene methyl glucoside is produced by addition polymerization of ethylene oxide to methyl glucoside. In accordance with the invention, commercially available products of polyoxyethylene methyl glucoside can be used. For example, such commercially available products include polyoxyethylene methyl glucoside (10E.O.) commercially available under the trade names of E-10 , 20 (Amachol Company) and the products NIKKOL BMG-10, -20 (Nikko Chemicals Company).

Polyoxypropylene methyl glucoside is produced by addition polymerization of propylene oxide to methyl glucoside. In accordance with the invention, commercially available products of polyoxypropylene methyl glucoside can be used. For example, such commercially available products include Glucam P-10, P-20 under trade names (Ikeda Corporation).

The amount of polyoxyethylene methyl glucoside and/or polyoxypropylene methyl glucoside is appropriately determined, depending on the amount of octyl methoxycinnamate to be blended. To the total amount of an external skin preparation, the amount is preferably at 1.0 to 20 % by weight, more preferably at 2.0 to 15 % by weight.

The dosage form of the external skin preparation of the invention is not limited but includes for example liquids, emulsions, gels, pastes and creams.

The external skin preparation of the invention is preferably used as a sun-screening cosmetic. The product form thereof is not limited. The term sun-screening cosmetic, means a cosmetic for protecting skin from ultraviolet rays.

The external skin preparation of the invention is produced by routine methods including blending the essential ingredients into formulation ingredients for external skin preparations (for example,oily matters,water,ethanol,humectants,surfactants, thickeners, metal sealing agents, chemicals, colors, and fragrance) and formulating the mixture into the intended dosage form.

The external skin preparation of the invention has an excellent effect of reducing the skin irritation of octyl methoxycinnamate and has good usability and a superior sun-screening effect.

### Examples

The invention is specifically described in the following examples. Amounts in blend are expressed in % by weight. Skin irritation was evaluated at the following test.
"Continuous skin irritation test"
Test method:
1. Coating 0.05 ml of a test solution on the dorsal part (2 × 2 cm) of a guinea pig (first coating).
2. Do a second coating 24 hours later.
3. Do a third coating another 24 hours later.
Evaluation method:
Before the second coating and the third coating and 24 hours after the third coating, skin reactions such as erythema and edema are observed under naked eyes . The evaluation is done on the following standards. Mean of three assessment scores is taken as skin irritation score.
Assessment standards of skin reactions

| Level of skin reactions | Assessment score |
|---|---|
| Absolutely no erythema observed | 0 |
| Slight erythema observed | 1 |
| Apparent erythema observed | 2 |
| Strong erythema or slight edema or crust observed | 3 |
| Apparent edema or crust observed | 4 |

The ingredients shown in "Table 1" were mixed together to prepare external skin preparations. Using the external skin preparations as test solutions, the test was done for evaluating irritation. Additionally, the amount of octyl methoxycinnamate is at 7.5 % by weight within the range never causing any skin irritation in the absence of titanium oxide and/or zinc oxide. As the fine particle zinc oxide, FINEX-50 with the surface treated with methyl hydrogen polysiloxane (mean particle size of 0.02 µm; manufactured by Sakai Chemical Industry Co., Ltd.) was used.

**Table 1**

| Ingredients | Amounts in blend (% by weight) | | |
|---|---|---|---|
| | Examples 1-4 Comparative Examples 3 - 19 | Comparative Example 1 | Comparative Example 2 |
| Fine particle zinc oxide (*1) | 20 | 20 | - |
| Octyl methoxycinnamate | 7.5 | 7.5 | 7.5 |
| Decamethylcyclopentasiloxane | qs. (25-39.4) | 40 | 60 |
| Methylpolysiloxane (*2) Dimethicone copolyol (*3) | 12 | 12 | 12 |
| | 0.5 | 0.5 | 0.5 |
| Water | 20 | 20 | 20 |
| Comparative blend ingredient | 0.5-15 | - | - |

| | | | |
|---|---|---|---|
| (*1) FINEX-50 (manufactured by Sakai Chemical Industry Co., Ltd.) | | | |
| (*2) Silicone KF-96 (manufactured by Shin-Etsu Chemical Co., Ltd.) | | | |
| (*3) Silicone SC 9450 (manufactured by Shin-Etsu Chemical Co., Ltd.) | | | |

The comparative blend ingredient is polyoxyethylene methyl glucoside in the Examples, which is blended as an irritation-reducing agent. In the Comparative Examples, the comparative blend ingredient is polyethylene glycol as the irritation-reducing agent described in Patent Reference 2 or other moisturizers. Herein, Comparative Example 1 is of a formulation with no blend of any comparative blend ingredient; and Comparative Example 2 is of a formulation in blend with neither any comparative blend ingredient nor zinc oxide powder. The comparative blend ingredient and the amount thereof are shown together with the test results in "Table 2". Fig.1 shows the results of "Table 2".

**Table 2**

| | Comparative blend ingredients and their amounts | Mean scores | Standard deviation |
|---|---|---|---|
| Example 1 | Polyoxyethylene methyl glucoside (*4) 2 % | 0.49 | 0.30 |
| Example 2 | Polyoxyethylene methyl glucoside (*4) 5 % | 0.39 | 0.14 |
| Example 3 | Polyoxyethylene methyl glucoside (*4) 10 % | 0.44 | 0.51 |
| Example 4 | Polyoxyethylene methyl glucoside (*4) 15 % | 0.44 | 0.51 |
| Comparative Example 1 | - | 1.10 | 0.40 |
| Comparative Example 2 Comparative Example 3 | - (zinc oxide powder removed) | 0.00 | 0.00 |
| | 1,3-butylene glycol 5 % | 0.83 | 0.35 |
| Comparative Example 4 | 1,3-butylene glycol 10 % | 1.11 | 0.51 |
| Comparative Example 5 | 1,3-butylene glycol 15% | 1.00 | 0.33 |
| Comparative Example 6 | Alkylene oxide derivative (*5) 5% | 1.11 | 0.38 |
| Comparative Example 7 | Alkylene oxide derivative (*5) 10% | 1.11 | 0.51 |
| Comparative Example 8 | Alkylene oxide derivative (*5) 15 % | 1.00 | 0.00 |
| Comparative Example 9 | Polyethylene glycol (MW 1500) 5 % | 1.00 | 0.30 |
| Comparative Example 10 | Polyethylene glycol (MW 1500) 10 % | 0.89 | 0.38 |
| Comparative Example 11 | Polyethylene glycol (MW 1500) 15 % | 0.78 | 0.19 |
| Comparative Example 12 | Vaseline 5 % | 0.78 | 0.27 |
| Comparative Example 13 | Vaseline 10 % | 0.78 | 0.19 |
| Comparative Example 14 | Vaseline 15 % | 0.78 | 0.19 |
| Comparative Example 15 | Sorbitol 5 % | 0.89 | 0.19 |
| Comparative Example 16 | Sorbitol 10 % | 0.89 | 0.19 |

| | | | |
|---|---|---|---|
| (*4)Glucam E-10 (Amachol Company) | | | |
| (*5) Polyoxyethylene • polyoxypropylene random polymer methyl ether | | | |

The above results indicate that the mean scores in the Examples are far smaller than those of the Comparative Examples in blend with polyethylene glycol as the irritation-reducing agent described in Patent Reference 2 and other humectants, indicating that the irritation-reducing effect of the invention' is very high in the Examples. Additionally, Comparative Examples 1 and 2 indicate that the skin irritation of octyl methoxycinnamate is never exerted in case of no zinc oxide powder blended and that the co-presence of both the two triggers the exertion of the skin irritation.

Other Examples of the invention are now described herein below. All are very excellent external skin preparations with the irritation-reducing effect and superior usability.

### Example 5 Sun-screening agent

| | |
|---|---|
| Octyl methoxycinnamate | 7.5 |
| Polypropylene glycol 1000 | 2.0 |
| tert-Butylmethoxydibenzoylmethane | 0.1 |
| Titanium dioxide | 5.0 |
| Decamethylcyclopentasiloxane | 30.0 |
| POE **•** methylpolysiloxane copolymer | 3.0 |
| Organo-modified montmorillonite | 0.8 |
| 1,3-Butylene glycol | 5.0 |
| Polyoxyethylene methyl glucoside (Glucam E-10™) | 3.0 |
| Preservative | appropriate amount |
| Fragrance | appropriate amount |
| Purified water | qs. |

### (Production method)

Individual ingredients were mixed together under agitation, to prepare a sun-screening agent.

### Example 6 Sun-screening emulsified foundation

| | |
|---|---|
| A. | |
| Purified water | 52.0 |
| Polyoxypropylene methyl glucoside (Glucam P-20™) | 5.0 |
| Ethanol | 7.0 |
| B. | |
| Talc | 7.0 |
| Titanium dioxide | 10.0 |
| Zinc oxide | 2.0 |
| Silicic anhydride | 2.0 |
| Nylon powder | 4.0 |
| Coloring pigment | 2.0 |
| C. | |
| Octyl methoxycinnamate | 15.0 |
| Tetraoctoate pentaerythrityl | 4.0 |
| Octamethylcyclotetrasiloxane | 10.0 |
| Rosin pentaerythrit ester | 1.5 |
| Diisooctoate neopentyl glycol | 5.0 |
| Triisooctoate glycerin | 2.0 |
| Polyoxyethylene modified dimethylpolysiloxane | 1.5 |
| Trimethylsiloxysilicic acid resin | 5.0 |

### (Production method)

After the ingredients described in A were agitated, the ingredients described in B were sufficiently mixed together and pulverized, and were then added to the agitated ingredients of A for treatment with a homomixer. After the ingredients described in C were dissolved and added to the resulting mixture for treatment with a homomixer, a sun-screening emulsified foundation was obtained.

### Example 7 Sun-screening agent

| | |
|---|---|
| Octyl methoxycinnamate | 7.5 |
| Polypropylene glycol 1000 | 2.0 |
| tert-Butylmethoxydibenzoylmethane | 0.1 |
| Titanium dioxide | 5.0 |
| Decamethylcyclopentasiloxane | 30.0 |
| POE **•** methylpolysiloxane copolymer | 3.0 |
| Organo-modified montmorillonite | 0.8 |
| 1,3-Butylene glycol | 5.0 |
| Polyoxyethylene methyl glucoside (Glucam E-10™) | 1.0 |
| Polyoxypropylene methyl glucoside (Glucam P-20™) | 2.0 |
| Preservative | appropriate amount |
| Fragrance | appropriate amount |
| Purified water | qs. |

### (Production method)

The individual ingredients were mixed together under agitation, to prepare a sun-screening agent.

## Claims

1. An external skin preparation comprising:
(1) octyl methoxycinnamate,
(2) oxide selected from the group consisting of titanium oxide, zinc oxide and mixtures thereof, and
(3) glucoside selected from the group consisting of polyoxyethylene methyl glucoside, polyoxypropylene methyl glucoside and mixture thereof.

2. An external skin preparation according to claim 1, which is a sun-screening cosmetic.

3. A method for reducing the skin irritation of octyl methoxycinnamate in an external skin preparation comprising:
(1) octyl methoxycinnamate,
(2) oxide selected from the group consisting of titanium oxide, zinc oxide and mixtures thereof, and
(3) glucoside selected from the group consisting of polyoxyethylene methyl glucoside, polyoxypropylene methyl glucoside and mixture thereof.

## Patentansprüche

1. Externes Hautpräparat, umfassend:
(1) Octylmethoxyzinnamat,
(2) Oxid, gewählt aus der Gruppe, bestehend aus Titanoxid, Zinkoxid und Mischungen hiervon, und
(3) Glucosid, gewählt aus der Gruppe, bestehend aus Polyoxyethylenmethylglucosid, Polyoxypropylenmethylglucosid und Mischungen hiervon.

2. Externes Hautpräparat nach Anspruch 1, das ein Sonnenschutzmittel-Kosmetikum ist.

3. Verfahren zur Verringerung der Hautirritation von Octylmethoxyzinnamat in einem externen Hautpräparat, umfassend:
(1) Octylmethoxyzinnamat,
(2) Oxid, gewählt aus der Gruppe, bestehend aus Titanoxid, Zinkoxid und Mischungen hiervon, und
(3) Glucosid, gewählt aus der Gruppe, bestehend aus Polyoxyethylenmethylglucosid, Polyoxypropylenmethylglucosid und Mischungen hiervon.

## Revendications

1. Préparation pour la peau à usage externe comprenant :
(1) du méthoxycinnamate d'octyle,
(2) un oxyde choisi dans le groupe constitué par l'oxyde de titane, l'oxyde de zinc et leurs mélanges et
(3) un glucoside choisi dans le groupe constitué par le glucoside de polyoxyéthylène méthyle, le glucoside de polyoxypropylène méthyle et un mélange de ceux-ci.

2. Une préparation pour la peau, à usage externe selon la revendication 1, qui est cosmétique formant un écran solaire.

3. Une méthode pour réduire l'irritation de la peau par le méthoxycinnamate d'octyle dans une préparation pour la peau à usage externe comprenant :
(1) du méthoxycinnamate d'octyle,
(2) un oxyde choisi dans le groupe constitué par l'oxyde de titane, l'oxyde de zinc et leurs mélanges et
(3) un glucoside choisi dans le groupe constitué par le glucoside de polyoxyéthylène méthyle, le glucoside de polyoxypropylène méthyle et un mélange de ceux-ci.
